# EUROPEAN PATENT SPECIFICATION

(11) **EP 4 572 736 B1**
(45) Date of publication and mention of the grant of the patent: **25.02.2026**
(21) Application number: 23726842.0
(22) Date of filing: 07.04.2023
(51) Int. Cl.: A61K 8/44, A61K 8/46, A61Q 19/10

(54) **PERSONAL CLEANSING COMPOSITION SUBSTANTIALLY FREE OF ALKYL SULFATE OR ALKYL ETHER SULFATE TYPE OF SURFACTANTS**
KÖRPERREINIGUNGSZUSAMMENSETZUNG, DIE IM WESENTLICHEN FREI VON TENSIDEN VOM TYP ALKYLSULFAT ODER ALKYLETHERSULFAT IST
COMPOSITION DE NETTOYAGE PERSONNEL SUBSTANTIELLEMENT EXEMPTE DE TENSIOACTIFS DE TYPE SULFATE D'ALKYLE OU SULFATE D'ÉTHER D'ALKYLE

(43) Date of publication of application: 25.06.2025
(73) Proprietor: The Procter & Gamble Company, Cincinnati, OH 45202 (US)
(72) Inventor: ZHANG, Lesheng, Beijing 101312 (CN); KATTAU, Nicole Lynette, Cincinnati Ohio 45202 (US)
(74) Representative: P&G Patent Germany
(86) International application number: PCT/CN2023/086792
(87) International publication number: WO 2024/207396

(56) References cited:
- EP-A1- 3 598 967
- WO-A1-2019/074992

## Description

### FIELD OF THE INVENTION

The present application generally relates to stable personal cleansing compositions with an improved rheology, their methods and their uses. The personal cleansing compositions comprise a surfactant system, wherein the surfactant system comprises a fatty acyl isethionate surfactant, a fatty acyl sarcosinate, a betaine and when the personal cleansing composition is substantially free of alkyl sulfate or alkyl ether sulfate type of surfactants.

### BACKGROUND OF THE INVENTION

Personal cleansing compositions have traditionally been marketed in a variety of forms such as bar soaps, creams, lotions, and gels. Typically, these products must satisfy a number of criteria to be acceptable to consumers. These criteria include cleansing effectiveness, skin feel, mildness to skin, hair, and ocular mucosae, and lather volume. Ideal personal cleansers should gently cleanse the skin or hair, cause little or no irritation, and should not leave the skin or hair overly dry after frequent use.

Anionic surfactants are widely used in personal cleansing compositions. Many of these anionic surfactants contain elongated micelles and are viscoelastic, which is of foremost importance, especially in the design of shampoos and body washes. In most personal cleansing compositions, alkyl sulfate or alkyl ether sulfate as the anionic surfactants predominate.

The formulation of environmentally friendly personal cleansing compositions is becoming a major challenge for satisfying a new expectation of consumers, in particular that of ecologically designed and/or natural products. It becomes necessary to propose personal cleansing compositions substantially free of alkyl sulfate and alkyl ether sulfate, which have good cosmetic qualities, mainly in terms of viscosity, creamy lather, and clean skin feel.

Consumers prefer sulfate-substantially free personal cleansing compositions due to perceived mildness and desirable sensorial experience. There is an interest to provide personal cleansing products that comprise alternative mild surfactant systems with relatively improved ecotoxic or ecologically friendly environmental profile.

Personal cleansing compositions having a surfactant system comprising a fatty acyl isethionate surfactant and being substantially free of alkyl sulfate or alkyl ether sulfate type of surfactants have been developed. Fatty acyl isethionates are mild anionic surfactants highly desirable in personal cleansing products for hair or skin, because fatty acyl isethionates can lather well, are mild to the skin and have good emollient properties.

However, personal cleansing composition comprising fatty acyl isethionates and fatty acyl sarcosinates may have phase stability challenges. Personal cleansing composition comprising fatty acyl isethionates may result in unstable personal cleansing compositions which can exhibit inconsistent rheology profiles. It has been observed phase separation of personal cleansing compositions comprising a fatty acyl isethionate and fatty acyl sarcosinate at 40°C after 3 months. The phase separation might be attributed to the presence of fatty acid that has been generated from the hydrolysis of the fatty acyl isethionate and fatty acyl sarcosinate. The initial wormlike and spherical micellar composition may separate into an upper lamellar phase and a lower micellar phase. Lamellar phases are typically relatively high ordered, surfactant-rich phases with relatively less water and low density such that the lamellar phases float.

Sulfate-substantially free personal cleansing compositions are also difficult to thicken sufficiently to afford the user good usage qualities. Two approaches are leveraged to attempt to thicken such formulas. One approach for instance is to use prominent levels of surfactants to benefit from the self-assembling properties of such ingredients. This approach is most common but it is also costly. The second approach for instance is to use elevated levels of rheology modifiers which can adversely impact the properties of the composition such as by decreasing the foam and ease of distribution of the composition. A third approach consists in using a mixture of a fatty alkanolamide such as cocamide MEA and a hydrophobically modified ethoxylated methyl glucoside such as PEG-120 methyl glucose trioleate to reach the desired viscosity of the personal cleansing composition, see for instance PCT/CN2022/086854. Reference is also made to EP3598967A1.

Hence, there is a need to provide a stable personal cleansing composition comprising a fatty acyl isethionate surfactant, being substantially free of alkyl sulfate or alkyl ether sulfate type of surfactants and having a satisfactory consistent rheology profile without the use of any polymeric rheology modifiers or increased levels of electrolyte.

Also, there remains a need for a personal cleansing composition, which is effective at cleaning even while containing lower number of active surfactants than typical cleansing products, but also still possesses good esthetic properties such as good foam, and is thick and creamy in texture, is silky to the touch and affords conditioning.

### SUMMARY OF THE INVENTION

A personal cleansing composition is provided and comprises: a) a surfactant system, wherein the surfactant system comprises: i) from 1.75% to 2.75%, preferably from 2.0% to 2.5%, more preferably from 2.25% to 2.5%, most preferably from 2.35% to 2.5%, of a fatty acyl isethionate surfactant by weight of the composition; ii) from 1.75% to 3.0%, preferably from 2.0% to 2.5%, more preferably from 2.25% to 2.5%, most preferably from 2.35% to 2.5%, of a fatty acyl sarcosinate surfactant by weight of the composition; iii) from 7.75% to 9.75%, preferably from 8.0% to 9.5%, more preferably from 8.75% to 9.5%, most preferably from 9.0% to 9.25% of a zwitterionic surfactant by weight of composition, wherein the zwitterionic surfactant comprises a betaine; b) wherein the pH is from 5.5 to 7, preferably from 5.6 to 6.5, more preferably from 5.65 to 6.0; and wherein the composition is substantially free of from alkyl sulfate and alkyl ether sulfate type of surfactants.

### DETAILED DESCRIPTION OF THE INVENTION

### Definitions of terms

In this document, including in all embodiments of all aspects of the present invention, the following definitions apply unless specifically stated otherwise.

All percentages are by weight (w/w) of the composition, unless otherwise specified. "% wt." means percentage by weight. References to 'parts' e.g. a mixture of 1 part X and 3 parts Y, is a ratio by weight. All ratios or percentages are weight ratios or weight percentages unless specifically stated otherwise.

An "active composition" is the composition absent water, and an "active ingredient" is the ingredient absent its water.

"QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about".

All measurements are understood to be made at 25°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR^{®} International.

Herein "min" means "minute" or "minutes". Herein "mol" means mole. Herein "g" following a number means "gram" or "grams". "Ex." means "example". All amounts as they pertain to listed ingredients are based on the active level and do not include carriers or byproducts that may be included in commercially available materials.

Herein, "comprising" means that other steps and other ingredients can be in addition. "Comprising" encompasses the terms "consisting of" and "consisting essentially of". The compositions, methods, uses, and processes of the present invention can comprise, consist of: and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

As used herein, the articles including "a" and "an" when used in a claim, are understood to mean "one or more" of what is claimed or described.

The terms "include," "includes," and "including," as used herein are meant to be nonlimiting.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the composition.

The term "free of" as used herein means that the composition comprises 0% of an ingredient by weight of the composition, thus no detectable amount of the stated ingredient.

The term "substantially free of" as used herein means less than 1.5%, less than 1.4%, less than 1.2%, less than 1%, less than 0.8%, less than 0.5%, less than 0.3%, or less than an immaterial amount of by weight of the composition.

Herein "Comp. Ex." or "C. Ex." means comparative example; and "Ex." means example.

The term "molecular weight" or "M.Wt." as used herein refers to the weight average molecular weight unless otherwise stated. The weight average molecular weight can be measured by gel permeation chromatography ("GPC").

The term "personal cleansing composition" as used herein refers to compositions intended for topical application to the hair and the skin, preferably to the skin, for cleansing.

The term "mixtures" as used herein is meant to include a simple combination of materials and any compounds that may result from their combination.

The term "molecular weight" or "M.Wt." as used herein refers to the weight average molecular weight unless otherwise stated. The weight average molecular weight can be measured by gel permeation chromatography ("GPC").

The term "room temperature" refers to a temperature of 25°C.

The term "rinse-off" as used herein means the intended product usage includes application to skin followed by rinsing and/or wiping the product from the skin within a few seconds to minutes of the application step. The product is generally applied and rinsed in the same usage event, for example, a shower or washing one's hands.

The term "derivative" as used herein refers to structures which are not shown but which one skilled in the art would understand are variations of the basic compound.

The methods as disclosed herein are cosmetic methods or non-therapeutic methods.

The objects of the present invention are to provide personal cleansing products, methods and uses of the products, the structures and the respective compositions as described in the Summary or as described hereinbelow for fulfilling the technical effects or goals as set out herein. These objects and other advantages as may be apparent to those skilled in the art can be achieved through the present invention, which is described in the above Summary of the Invention and Detailed Description of the invention and which is defined in the claims which follow.

### BENEFITS

Personal cleansing compositions comprising a fatty acyl isethionate and a fatty acyl sarcosinate have shown phase stability challenges. Such phase separation is typically caused by the accumulation of fatty acid which is generated by the hydrolysis of the fatty acyl isethionate and fatty acyl sarcosinate overtime.

Phase separation has been observed for personal cleansing compositions comprising a fatty acyl isethionate at 40°C after 3 months (see C. Ex. 1 for an example). The mechanism of the phase separation might lie in the hydrolysis of the fatty acyl isethionate and other anionic surfactants such as a fatty acyl sarcosinate catalyzed in an acidic medium (e.g. pH at 5).

Increasing the pH would slow down the hydrolysis kinetics (see C. Ex. 2 for an example), however, at a neutral pH, the zero-shear viscosity of the personal cleansing composition may collapse. Only a small portion of a deprotonated form of the anionic surfactant, e.g. fatty acyl isethionate or fatty acyl sarcosinate, is needed to form a stable wormlike micellar phase without the addition of any electrolyte. The pH of the personal cleansing composition cannot be raised far from the pKa of the anionic surfactant, otherwise, the viscosity peak is missed and the personal cleansing composition cannot meet the rheologic profile.

As there is a need to increase the pH to control the hydrolysis kinetics of the fatty acyl isethionate, a new combination of ingredients was needed to build the consistent viscosity of the personal cleansing composition (See Ex. 1 and Ex. 2 as suitable examples).

It has been found that the personal cleansing composition needs to include a surfactant system, wherein the surfactant system comprises: i) from 1.75% to 2.75%, preferably from 2.0% to 2.5%, more preferably from 2.25% to 2.5%, most preferably from 2.35% to 2.5%, of a fatty acyl isethionate surfactant by weight of the composition; ii) from 1.75% to 3.0%, preferably from 2.0% to 2.5%, more preferably from 2.25% to 2.5%, most preferably from 2.35% to 2.5%, of a fatty acyl sarcosinate surfactant by weight of the composition; iii) from 7.75% to 9.75%, preferably from 8.0% to 9.5%, more preferably from 8.75% to 9.5%, most preferably from 9.0% to 9.25% of a zwitterionic surfactant by weight of composition, wherein the zwitterionic surfactant comprises a betaine; b) wherein the pH is from 5.5 to 7, preferably from 5.6 to 6.5, more preferably from 5.65 to 6.0; and wherein the composition is substantially free of from alkyl sulfate and alkyl ether sulfate type of surfactants.

Hence, the surfactant system can be optimized to enable building the consistent viscosity of the personal cleansing composition. The personal cleansing composition is stable and shows a transparent homogeneous appearance overtime, through the shelf life.

### SURFACTANT SYSTEM

A personal cleansing composition is provided and comprises a surfactant system, wherein the surfactant system comprises: i) from 1.75% to 2.75%, preferably from 2.0% to 2.5%, more preferably from 2.25% to 2.5%, most preferably from 2.35% to 2.5%, of a fatty acyl isethionate surfactant by weight of the composition; ii) from 1.75% to 3.0%, preferably from 2.0% to 2.5%, more preferably from 2.25% to 2.5%, most preferably from 2.35% to 2.5%, of a fatty acyl sarcosinate surfactant by weight of the composition; iii) from 7.75% to 9.75%, preferably from 8.0% to 9.5%, more preferably from 8.75% to 9.5%, most preferably from 9.0% to 9.25% of a zwitterionic surfactant by weight of composition, wherein the zwitterionic surfactant comprises a betaine; b) wherein the pH is from 5.5 to 7, preferably from 5.6 to 6.5, more preferably from 5.65 to 6.0.

The personal cleansing composition is substantially free of alkyl sulfate and/or alkyl ether sulfate type of surfactant. Namely, the personal cleansing composition comprises less than 1.5%, or less than 1.4%, or less than 1.2%, or less than 1%, or less than 0.8%, or less than 0.5%, or less than 0.3%, or is free of alkyl sulfate and/or alkyl ether sulfate type of surfactant by weight of the composition.

Preferably, the personal cleansing composition comprises less than 1.5%, or less than 1.4%, or less than 1.2%, or less than 1%, or less than 0.8%, or less than 0.5%, or less than 0.3%, or is free of any alkyl sulfate which comprises C₁₂-C₁₈ alkyl sulfate and/or any alkyl ether sulfate including alkyl glyceryl ether sulfates.

More preferably, the personal cleansing composition comprises less than 1.5%, or less than 1.4%, or less than 1.2%, or less than 1%, or less than 0.8%, or less than 0.5%, or less than 0.3%, or is free of sodium lauryl sulfate.

Alternatively, the personal cleansing composition is free of alkyl sulfate and/or alkyl ether sulfate type of surfactant. Namely, the personal cleansing composition comprises 0% of alkyl sulfate and/or alkyl ether sulfate type of surfactant by weight of the composition, thus no detectable amount of alkyl sulfate and/or alkyl ether sulfate type of surfactant.

In that respect, the personal cleansing composition may not comprise any alkyl sulfate which comprises C₁₂-C₁₈ alkyl sulfate and/or any alkyl ether sulfate including alkyl glyceryl ether sulfates.

The personal cleansing composition may not comprise any alkyl ether sulfates which are those having the formula:

RO(CH₂CH₂O)ₙSO₃M

wherein R is an alkyl or alkenyl having 8 to 18 carbons, preferably 12 to 18 carbons, n has an average value of greater than at least 0.5, preferably between 2 and 3; and M is a solubilizing cation such as sodium, potassium, ammonium or substituted ammonium.

The personal cleansing composition may not comprise any ammonium and sodium lauryl ether sulfates.

If the personal cleansing composition does contain alkyl sulfate and/or alkyl ether sulfate type of surfactant, its content of such a weight proportion of: alkyl sulfates or alkyl ether sulfate type surfactant is less than or equal to the sum of 0.6, more preferably less than or equal to the sum of 0.2, even more preferably equal to 0.

### Fatty acyl isethionate

The fatty acyl isethionate surfactant may be defined as an isethionate according to the general Formula (I): 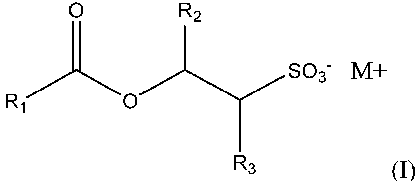 wherein R₁ is a saturated or unsaturated, straight or branched, alkyl or alkenyl chain with from 6 to 30 carbon atoms, preferably from 8 to 22 carbon atoms, more preferably from 9 to 18 carbon atoms, R₂ and R₃ are each independently H or (C₁-C₄) alkyl, and M⁺ is an alkali metal, preferably lithium, sodium, potassium; or M⁺ is an alkali-earth metal, preferably magnesium; or M⁺ is an ammonium or a substituted ammonium cation.

Preferably, R₁ may be a saturated or unsaturated, straight or branched alkyl or alkenyl, preferably an alkyl chain with from 6 to 30 carbon atoms, preferably from 8 to 22 carbon atoms, more preferably from 9 to 18 carbon atoms, R₂ and R₃ are H, and M⁺ is an alkali metal, preferably sodium, potassium; or M⁺ is an ammonium cation.

More preferably, R₁ may be a saturated or unsaturated, straight or branched alkyl chain with from 9 to 18 carbon atoms, R₂ and R₃ are H, and M⁺ is sodium or an ammonium cation.

Suitable fatty acyl isethionate surfactants may include the reaction product of fatty acids esterified with isethionic acid and neutralized with sodium hydroxide. Suitable fatty acids for isethionate surfactants can be derived from coconut oil or palm kernel oil, for instance. Additional examples of suitable isethionic anionic surfactants are described in U.S. Pat. No. 2,486,921; U.S. Pat. No. 2,486,922; and U.S. Pat. No. 2,396,278,

The personal cleansing composition comprises a surfactant system. The surfactant system comprises from 1.75% to 2.75%, preferably from 2.0% to 2.5%, more preferably from 2.25% to 2.5%, most preferably from 2.35% to 2.5%, of a fatty acyl isethionate surfactant by weight of the composition. The concentrations mentioned here are total concentration ranges in case more than one fatty acyl isethionate surfactant is present. The specified ranges are provided by weight and relate to the total weight of the personal cleansing composition.

The fatty acyl isethionate surfactant may be selected from the group consisting of sodium lauroyl isethionate, sodium lauroyl methyl isethionate, sodium oleoyl isethionate, sodium oleoyl methyl isethionate, sodium stearoyl isethionate, sodium stearoyl methyl isethionate, sodium myristoyl isethionate, sodium myristoyl methyl isethionate, sodium palmitoyl isethionate, sodium palmitoyl methyl isethionate, sodium cocoyl isethionate, sodium cocoyl methyl isethionate, a blend of stearic acid and sodium cocoyl isethionate, ammonium cocoyl isethionate, ammonium cocoyl methyl isethionate, and mixtures thereof.

The fatty acyl isethionate surfactant may be preferably selected from the group consisting of sodium lauroyl isethionate, sodium myristoyl isethionate, sodium palmitoyl isethionate, sodium stearoyl isethionate, sodium oleoyl isethionate, sodium cocoyl isethionate, ammonium cocoyl isethionate, and mixtures thereof.

The fatty acyl isethionate surfactant may be more preferably selected from the group consisting of sodium lauroyl isethionate, sodium cocoyl isethionate, ammonium cocoyl isethionate, and mixtures thereof.

The fatty acyl isethionate surfactant may be even more preferably selected from the group consisting of sodium cocoyl isethionate, ammonium cocoyl isethionate, and mixtures thereof.

The fatty acyl isethionate surfactant may most preferably comprise sodium cocoyl isethionate.

In that aspect, the personal cleansing composition may comprise a surfactant system, wherein the surfactant system comprises: from 1.75% to 2.75%, preferably from 2.0% to 2.5%, more preferably from 2.25% to 2.5%, most preferably from 2.35% to 2.5%, of sodium cocoyl isethionate by weight of the composition.

Corresponding commercial products are available, for example, from the company Innospec under the trade name "Iselux^{®}" and from Clariant or Uniquema under the trade names "Hostapon^{®}" or "Arlatone^{®}". Examples of other commercial fatty acyl isethionates that may be used can be Hostapon^{®} surfactants from Clariant such as for sodium cocoyl isethionate: Hostapon^{®} SCI-85C, Hostapon^{®} SCI-78C, or a blend of stearic acid with sodium cocoyl isethionate: Hostapon^{®} SCI-65C. Examples of other commercial fatty acyl isethionates that may be used can be "Jordapon^{®}" surfactants from BASF such as Jordapon^{®} CI prill or Jordapon^{®} CI65; and sodium cocoyl isethionate from Yongan Daily Chemical Co. such as YA-SCI-85^{®} or YA-SCI-65^{®}.

Fatty acyl isethionates surfactants are typically prepared by the reaction of an isethionate salt such as metal or ammonium isethionate and an a saturated or unsaturated, straight or branched, alkyl or alkenyl chain fatty acid having from 6 to 30 carbon atoms, preferably from 8 to 22 carbon atoms, more preferably from 6 to 18 carbon atoms. Depending on the processing conditions used, the resulting fatty acyl isethionate surfactant can be a mixture of 45 to 95% by weight of fatty acyl isethionates and 0 to 40 wt.% of free fatty acids, in addition to isethionates salts, typically less than 5 wt.%, and trace (less than 2 wt.%) of other impurities, by total weight of the resulting fatty acyl isethionate surfactant. A mixture of aliphatic fatty acids may be used for the preparation of commercial fatty acyl isethionates surfactants.

### Fatty acyl sarcosinate

The surfactant system further comprises from 1.75% to 3.0%, preferably from 2.0% to 2.5%, more preferably from 2.25% to 2.5%, most preferably from 2.35% to 2.5%, of a fatty acyl sarcosinate surfactant by weight of the composition.

The fatty acyl sarcosinate surfactant may be a sarcosinate according to the general Formula (II): 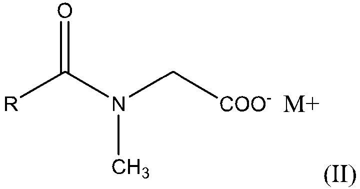 wherein R is a saturated or unsaturated, straight or branched or alkenyl, preferably alkyl chain with 7 to 17 carbon atoms, preferably with 9 to 13 carbon atoms and M⁺ is H, a sodium, potassium or ammonium cation.

The fatty acyl sarcosinate may be selected from the group consisting of sodium lauroyl sarcosinate, sodium cocoyl sarcosinate, sodium myristoyl sarcosinate, TEA-cocoyl sarcosinate, ammonium cocoyl sarcosinate, ammonium lauroyl sarcosinate, dimer dilinoleyl bis-lauroyl glutamate/lauroyl sarcosinate, lauroyl sarcosinate, isopropyl lauroyl sarcosinate, potassium cocoyl sarcosinate, potassium lauroyl sarcosinate, sodium oleoyl sarcosinate, sodium palmitoyl sarcosinate, TEA-lauroyl sarcosinate, TEA-oleoyl sarcosinate, TEA-palm kernel sarcosinate, and mixtures thereof. For instance, TEA-cocoyl sarcosinate is the triethanolamine salt of cocoyl sarcosine.

Preferably, the fatty acyl sarcosinate may be selected from the group consisting of sodium lauroyl sarcosinate, sodium myristoyl sarcosinate, sodium cocoyl sarcosinate, and mixtures thereof.

The fatty acyl sarcosinate may most preferably comprise sodium lauroyl sarcosinate.

In that aspect, the personal cleansing composition may comprise a surfactant system, wherein the surfactant system comprises: from 1.75% to 3.0%, preferably from 2.0% to 2.5%, more preferably from 2.25% to 2.5%, most preferably from 2.35% to 2.5%, of sodium lauroyl sarcosinate by weight of the composition.

By decreasing slightly the level of the fatty acyl sarcosinate in the personal cleansing composition, a consistent rheology profile could be obtained.

The weight ratio of the fatty acyl sarcosinate to the fatty acyl isethionate may be above 0.5:1, or from 1.75:1 to 1:0.65, preferably from 1.25:1 to 1:0.75, more preferably from 1.2:1 to 1:0.95.

The composition may comprise sodium lauroyl sarcosinate and sodium cocoyl isethionate at a weight ratio sarcosinate to isethionate above 0.5:1, or from 1.75:1 to 1:0.65, preferably from 1.25:1 to 1:0.75, more preferably from 1.2:1 to 1:0.95.

In that aspect, by selecting the respective weight ratio, it is possible to enable building and improving the rheology profile of the personal cleansing composition even further.

### Additional anionic surfactant

The composition may comprise one or more additional anionic surfactant not being a fatty acyl isethionate or a fatty acyl sarcosinate.

The surfactant system may comprise from 0.5% to 25%, preferably from 1% to 20%, more preferably from 5% to 15% of the one or more additional anionic surfactant not being a fatty acyl isethionate or a fatty acyl sarcosinate by weight of the composition.

The one or more additional anionic surfactant not being a fatty acyl isethionate or a fatty acyl sarcosinate may be selected from the group consisting of sulfosuccinates, sulfonates, sulfoacetates, acyl glycinates, acyl alaninates, acyl glutamates, lactates, lactylates, taurates, and mixtures thereof.

Non-limiting examples of sulfosuccinate surfactants can include disodium N-octadecyl sulfosuccinate, disodium lauryl sulfosuccinate, diammonium lauryl sulfosuccinate, sodium lauryl sulfosuccinate, disodium laureth sulfosuccinate, tetrasodium N-(1,2-dicarboxyethyl)-N-octadecyl sulfosuccinnate, diamyl ester of sodium sulfosuccinic acid, dihexyl ester of sodium sulfosuccinic acid, dioctyl esters of sodium sulfosuccinic acid, and combinations thereof.

Non-limiting examples of sulfonates can include alpha olefin sulfonates, linear alkylbenzene sulfonates, sodium laurylglucosides hydroxypropylsulfonate, and combinations thereof.

Non-limiting examples of sulfoacetates can include sodium lauryl sulfoacetate, ammonium lauryl sulfoacetate, and combination thereof.

Non-limiting examples of acyl glycinates can include sodium cocoyl glycinate, sodium lauroyl glycinate, and combination thereof.

Non-limiting example of acyl alaninates can include sodium cocoyl alaninate, sodium lauroyl alaninate, sodium N-dodecanoyl-1-alaninate, and combinations thereof.

Non-limiting examples of acyl glutamates can be selected from the group consisting of sodium cocoyl glutamate, disodium cocoyl glutamate, ammonium cocoyl glutamate, diammonium cocoyl glutamate, sodium lauroyl glutamate, disodium lauroyl glutamate, sodium cocoyl hydrolyzed wheat protein glutamate, disodium cocoyl hydrolyzed wheat protein glutamate, potassium cocoyl glutamate, dipotassium cocoyl glutamate, potassium lauroyl glutamate, dipotassium lauroyl glutamate, potassium cocoyl hydrolyzed wheat protein glutamate, dipotassium cocoyl hydrolyzed wheat protein glutamate, sodium capryloyl glutamate, disodium capryloyl glutamate, potassium capryloyl glutamate, dipotassium capryloyl glutamate, sodium undecylenoyl glutamate, disodium undecylenoyl glutamate, potassium undecylenoyl glutamate, dipotassium undecylenoyl glutamate, disodium hydrogenated tallow glutamate, sodium stearoyl glutamate, disodium stearoyl glutamate, potassium stearoyl glutamate, dipotassium stearoyl glutamate, sodium myristoyl glutamate, disodium myristoyl glutamate, potassium myristoyl glutamate, dipotassium myristoyl glutamate, sodium cocoyl/hydrogenated tallow glutamate, sodium cocoyl/palmoyl/sunfloweroyl glutamate, sodium hydrogenated tallowoyl glutamate, sodium olivoyl glutamate, disodium olivoyl glutamate, sodium palmoyl glutamate, disodium palmoyl glutamate, TEA-cocoyl glutamate, TEA-hydrogenated tallowoyl glutamate, TEA-lauroyl glutamate, and mixtures thereof.

Non-limiting example of lactates can include sodium lactate.

Non-limiting examples of lactylates can include sodium lauroyl lactylate, sodium cocoyl lactylate, and combination thereof.

Non-limiting examples of acyl taurates can include sodium methyl cocoyl taurate, sodium methyl lauroyl taurate, sodium methyl oleoyl taurate, and combinations thereof.

In that case, alkyl is defined as a saturated or unsaturated, straight or branched alkyl chain with 6 to 30 carbon atoms, preferably with 8 to 22 carbon atoms, more preferably with 9 to 18 carbon atoms. In that case, acyl is defined as of formula R-C(O)-, wherein R is a saturated or unsaturated, straight or branched alkyl or alkenyl, preferably alkyl chain with 6 to 30 carbon atoms, preferably with 8 to 22 carbon atoms, more preferably with 9 to 18 carbon atoms.

### Zwitterionic surfactant

The surfactant system comprises from 7.75% to 9.75%, preferably from 8.0% to 9.5%, more preferably from 8.75% to 9.5%, most preferably from 9.0% to 9.25% of a zwitterionic surfactant by weight of composition, wherein the zwitterionic surfactant comprises a betaine.

The zwitterionic surfactant may comprise an alkyl betaine or an alkyl amidopropyl betaine; or a sulfobetaine.

Examples of betaine zwitterionic surfactants may include coco dimethyl carboxymethyl betaine, cocoamidopropyl betaine (CAPB), coco-betaine, lauryl amidopropyl betaine (LAPB), oleyl betaine, lauryl dimethyl carboxymethyl betaine, lauryl dimethyl alpha-carboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyethyl betaine, and mixtures thereof.

Examples of sulfobetaines may include coco dimethyl sulfopropyl betaine, stearyl dimethyl sulfopropyl betaine, lauryl dimethyl sulfoethyl betaine, lauryl bis-(2-hydroxyethyl) sulfopropyl betaine and mixtures thereof.

More preferably, the zwitterionic surfactant may be selected from the group consisting of cocamidopropyl betaine, lauramidopropyl betaine, coco betaine, and mixtures thereof.

The surfactant system may comprise from 7.75% to 9.75%, preferably from 8.0% to 9.5%, more preferably from 8.75% to 9.5%, most preferably from 9.0% to 9.25% of cocamidopropyl betaine.

In that aspect, the phase stability of the personal cleansing composition can be further enhanced.

The weight ratio of betaine to fatty acyl sarcosinate surfactant is above 2.5:1, or from 3.4:1 to 3.8:1, preferably from 3.45:1 to 3.7:1, more preferably from 3.5:1 to 3.65:1.

The composition may comprise cocamidopropyl betaine and sodium lauroyl sarcosinate at a weight ratio above 2.5:1, or from 3.4:1 to 3.8:1, preferably from 3.45:1 to 3.7:1, more preferably from 3.5:1 to 3.65:1.

The weight ratio of betaine to fatty acyl sarcosinate surfactant can help to further enhance the rheology profile of the personal cleansing composition systematically. The weight ratio of betaine to fatty acyl sarcosinate surfactant has been optimized to enable building the viscosity of the personal cleansing composition at the recited pH.

### Additional co-surfactant

The surfactant system may include an additional co-surfactant, wherein the additional co-surfactant comprises an amphoteric surfactant. Suitable amphoteric surfactants can include those described in U.S. Patent No. 5,104,646 and U.S. Patent No. 5,106,609,

Amphoteric surfactants can include those that can be broadly described as derivatives of aliphatic secondary and tertiary amines in which an aliphatic radical can be straight or branched chain and wherein an aliphatic substituent can contain from 8 to 18 carbon atoms such that one carbon atom can contain an anionic water solubilizing group, e.g., carboxy, sulfonate, phosphate, or phosphonate. Examples of compounds falling within this definition can be sodium 3-dodecyl-aminopropionate, sodium 3-dodecylaminopropane sulfonate, N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No. 2,438,091, and products described in U.S. Patent No. 2,528,378,

The amphoteric surfactant included in the personal cleansing composition described herein may be preferably selected from the group consisting of sodium lauroamphoacetate, sodium cocoamphoacetate, disodium lauroamphoacetate, disodium cocodiamphoacetate, and mixtures thereof.

### pH

The pH of the personal cleansing composition is from 5.5 to 7, preferably from 5.5 to 6.5, more preferably from 5.65 to 6.0. Increasing the pH can help for preventing hydrolysis of the fatty acyl isethionate and the fatty acyl sarcosinate. pH may be measured according to the Product pH Measurement Test Method, described hereafter.

A variety of compounds may be used to adjust the pH value of a composition. Such suitable compounds can include, but are not limited to, citric acid, acetic acid, hydrochloric acid, sodium hydroxide, magnesium hydroxide, triethylamine, diethylamine, ethylamine, monoethanol amine, and any mixtures thereof. The personal cleansing composition may comprise greater than 0% to 2% of the pH adjusting agent by weight of the composition, preferably wherein the pH adjusting agent comprises citric acid.

By rising up the pH of the personal cleansing composition, the kinetics of generating fatty acids from the hydrolysis of the fatty acyl isethionate or the fatty acyl sarcosinate is slowed down. Increasing the pH of the personal cleansing composition can help to prevent phase separation of the personal cleansing composition. Then, the surfactant levels and/or can be optimized as described herein for building and improving the rheology profile of the personal cleansing composition.

### Electrolyte

The personal cleansing composition may further comprise from 0.05 to 5%, preferably from 1% to 5%, more preferably from 2% to 5%, even more preferably from 2.75% to 4% of an electrolyte by weight of the composition. The addition of an electrolyte can help to elongate the micelles of the surfactant system and to improve further the viscosity of the composition if needed.

The electrolyte may be selected from the group of sodium or potassium citrate, calcium chloride, calcium bromide, zinc chloride, barium chloride, calcium nitrate, potassium chloride, sodium chloride, potassium iodide, sodium bromide, ammonium bromide, sodium sulfate, and mixtures thereof.

The electrolyte may be preferably selected from the group of sodium or potassium citrate, calcium chloride, potassium chloride, sodium chloride, and mixtures thereof.

Most preferably, the personal cleansing composition may further comprise from 3% to 4% of an electrolyte by weight of the composition, wherein the electrolyte is sodium chloride.

The electrolyte itself can increase the initial viscosity, however, the inventors have found that the rheology profile of the personal cleansing composition could be optimized at a constant level of an electrolyte (4 wt.% sodium chloride) or even without any addition of an electrolyte by adjusting the surfactant levels and/or their weight ratios.

The personal cleansing composition may not comprise any rheology polymeric modifiers Examples of rheology polymeric modifiers may be but not limited to: sodium polyacrylate, acrylates copolymer, Acrylates/Vinyl Isodecanoate Crosspolymer, Acrylates/C10-30 Alkyl Acrylate Crosspolymer, Acrylates/C10-30 alkyl acrylate crosspolymer comprising stearyl side chains with less than about 1% Hydrophobic modification, acrylates/C10-30 alkyl acrylate crosspolymer including octyl side chains with less than about 5% Hydrophobic modification, Ammonium Acryloyldimethyltaurate/Beheneth-25 Methacrylate Crosspolymer, Acrylates/Beheneth-25 Methacrylate Copolymer, Acrylates/Steareth-20 Methacrylate Copolymer, and Acrylates/Steareth-20 Methacrylate Crosspolymer, PEG-150/Decyl Alcohol/SMDI Copolymer, PEG-150/stearyl alcohol/SMDI copolymer, hydroxypropyl starch phosphate, distarch phosphate, sodium carboxymethyl starch, hydroxypropyl starch phosphate, starch, Tapioca starch, xanthan gum, gellan gum, carboxymethyl cellulose, carboxymethyl hydroxyethyl cellulose, hydroxypropyl methyl cellulose, guar gum, hydroxypropyl guar, sodium alginate, and mixtures thereof.

The personal cleansing composition may not comprise any cationic deposition polymers. Nonlimiting examples of cationic deposition polymers for use in the personal care composition include cationic cellulose derivatives. Cationic cellulose polymers are the salts of hydroxyethyl cellulose reacted with trimethyl ammonium substituted epoxide, referred to in the industry (CTFA) as Polyquaternium 10 which are available from Amerchol Corp. (Edison, N.J., USA) in their Polymer KG, JR and LR series of polymers, e.g. KG-30M. Other suitable cationic deposition polymers include cationic guar gum derivatives, such as guar hydroxypropyltrimonium chloride, specific examples of which include the Jaguar series (preferably Jaguar C-17) commercially available from Rhodia Inc., and N-Hance polymer series commercially available from Aqualon.

The personal cleansing composition may not comprise a direct dye and/or any oxidative dye precursors.

### Fragrance Component

The personal cleansing composition may further comprise from 0.01% to 2% of a fragrance component by weight of the composition, preferably from 0.1% to 1.75% of a fragrance component by weight of the composition, more preferably from 0.5% to 1.6% of a fragrance component by weight of the composition, even more preferably from 0.8% to 1.0% of a fragrance component by weight of the composition.

Typically the fragrance component may be a blend of perfumes and aroma chemicals. As used herein, "fragrance" is used to indicate any odoriferous material.

A wide variety of chemicals are known as fragrances, including alcohols, aldehydes, ketones, and esters. Non-limiting examples of the fragrances useful herein include pro-fragrances such as acetal pro-fragrances, ketal pro-fragrances, ester pro-fragrances, hydrolyzable inorganicorganic pro-fragrances, and mixtures thereof. The fragrances may be released from the pro-fragrances in a number of ways. For example, the fragrance may be released as a result of simple hydrolysis, or by a shift in an equilibrium reaction, or by a pH-change, or by enzymatic release. The fragrances herein may be relatively simple in their chemical make-up, comprising a single chemical, or may comprise highly sophisticated complex mixtures of natural and synthetic chemical components, all chosen to provide any desired odor.

The fragrances may have a boiling point (BP) of 500°C or lower, 400°C or lower, or 350°C or lower. The BP of many fragrances are disclosed in Perfume and Flavor Chemicals (Aroma Chemicals), Steffen Arctander (1969). The ClogP value of the fragrances may be 0.1 or greater, 0.5 or greater, 1.0 or greater, and 1.2 or greater. As used herein, "ClogP" means the logarithm to the base 10 of the octanol/water partition coefficient. The ClogP may be readily calculated from a program called "CLOGP" which is available from Daylight Chemical Information Systems Inc., Irvine Calif., USA. Octanol/water partition coefficients are described in more detail in U.S. Patent No. 5,578,563,

Suitable fragrances are also disclosed in U.S. Patent No. 4,145,184, U.S. Patent No. 4,209,417, U.S. Patent No. 4,515,705, and U.S. Patent No. 4,152,272, Non·-limiting examples of fragrances include animal fragrances such as musk oil, civet, castoreum, ambergris, plant fragrances such as nutmeg extract, cardomon extract, ginger extract, cinnamon extract, patchouli oil, geranium oil, orange oil, mandarin oil, orange Hower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

Other examples of suitable fragrances include, but are not limited to, chemical substances such as acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, borneol, butyl acetate, camphor, carbitol, cinnamaldehyde, cinnamyl acetate, cinnamyl alcohol, cis-3-hexanol and ester derivatives, cis-3-bexenyl methyl carbonate, citral, citronnellol and ester derivatives, cumin aldehyde, cyclamen aldehyde, cyclogalbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltoiide, fenchone, fruity esters such as ethyl 2-methyl butyrate, galaxolide, geraniol and ester derivatives, helional, 2-heptonone, hexenol, α-hexylcinnamic aldehyde, hydroxycitronellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dibydrojasmonate, methyl eugenol, methyl ionone, methyl-α-naphthyl ketone, methylphenylcarbinyl acetate, mugetanol, γ-nonalactone, octanal, phenyl ethyl acetate, phenylacetaldehyde dimethyl acetate, phenoxyethyl isobutyrate, phenyl ethyl alcohol, pinenes, sandalore, santaiol, stemone, thymol, terpenes, triplal, triethyl citrate, 3,3,5-trimethylcyclohexanol, γ-undecalactone, undecenal, vanillin, veloutone, verdox, and mixtures thereof.

In the personal cleansing composition, the fragrance component may comprise ketone and/or aldehyde fragrance components. In addition, the fragrance component may further comprise any fragrances as set out just above.

Ketone fragrance components may be selected from alicyclic ketones such a β-ionone, terpene ketones such as l-carvone, and macrocyclic ketones such as cyclopentadecanone.

Aldehyde fragrance components may be selected from fatty aldehydes such as 2,6-nonadienal, terpene aldehydes such as citral, and aromatic aldehydes such as α-hexylcinnamic aldehyde, cinnamaldehyde.

Thus, preferably the fragrance component may comprise ketone and/or aldehyde fragrance components, wherein the ketone and/or aldehyde fragrance components may be selected from the group consisting of acetophenone, adoxal, aldehyde C-12, aldehyde C-14, aldehyde C-18, α-amylcinnamic aldehyde, anisaldehyde, benzaldehyde, camphor, cinnamaldehyde, citral, cumin aldehyde, cyclamen aldehyde, damascones, fenchone, helional, 2-heptonone, α-hexylcinnamic aldehyde, hydroxycitronellal, ionones, lilial, lyral, melonal, p-methylacetophenone, methyl cedrylone, methyl ionone, methyl-α-naphthyl ketone, γ-nonalactone, octanal, phenylacetaldehyde dimethyl acetate, triplal, γ-undecalactone, undecenal, vanillin, veloutone, and mixtures thereof.

### Preservative

The personal cleansing composition may comprise from 0.01% to 1.0%, preferably from 0.02% to 0.4%, more preferably from 0.05% to 0.2%, most preferably from 0.05% to 0.1% of a preservative by weight of the composition.

The preservative may include a salicylate salt and a benzoate salt, wherein a total amount of the salicylate salt and the benzoate salt is from 0.2% to 0.9%, preferably from 0.5% to 0.85%, more preferably from 0.75% to 0.85%, by weight of the composition.

The weight ratio of the salicylate salt to the benzoate salt may be from 1:1.10 to 1:1.20, preferably from 1:1.125 to1:1.175.

The salicylate salt may be sodium salicylate. The benzoate salt may be sodium benzoate.

### OPTIONAL INGREDIENTS

As can be appreciated, the compositions described herein may include a variety of optional components to tailor the properties and characteristics of the composition. As can be appreciated, suitable optional components are well known and can generally include any components which are physically and chemically compatible with the essential components of the compositions described herein. Optional components should not otherwise unduly impair product stability, aesthetics, or performance. Individual concentrations of optional components can generally range from 0.001% to 10%, by weight of the composition. Optional components can be further limited to components which will not impair the clarity of a translucent composition.

Still, the personal cleansing composition may not include or may be free of direct dyes, oxidative dyes, parabens, or mixtures thereof.

Optional components may include, but are not limited to, conditioning agents (including hydrocarbon oils, fatty esters, silicones), anti-dandruff actives, and chelating agents. Additional suitable optional ingredients include but are not limited to particles, anti-microbials, foam boosters, anti-static agents, moisturizing agents, propellants, self-foaming agents, pearlescent agents, opacifiers, sensates, suspending agents, solvents, diluents, anti-oxidants, vitamins, and mixtures thereof.

### METHOD

A method of building a consistent viscosity of a stable personal cleansing composition is provided and comprises the step of forming a personal cleansing composition as set out hereinbefore.

### FORMS AND USES

### Product Form

The personal cleansing composition may be presented in typical personal cleansing formulations. They may be in the form of solutions, dispersion, emulsions, foams, and other delivery mechanisms. The personal cleansing composition may be a rinse-off composition.

The personal cleansing composition may be extrudable or dispensable from a single chamber package. The personal cleansing compositions can be in the form of liquid, semi-liquid, cream, lotion or gel, or solid compositions intended for topical application to skin.

Examples of personal cleansing compositions can include but are not limited to body wash, moisturizing body wash, foaming body wash, shower gels, a shower or bath cream, skin cleansers, cleansing milks, body wash, in shower body moisturizer, gel, emulsion, oil, mousse or spray.

The personal cleansing composition may be not in the form of a liquid hand wash or a liquid hand sanitizer.

The product forms contemplated for purposes of defining the personal cleansing compositions and methods are rinse-off formulations by which it is meant that the product is applied topically to the skin and then subsequently (i.e., within minutes) rinsed away with water, or otherwise wiped off using a substrate or other suitable removal means.

### Uses

The personal cleansing composition as set out hereinabove may be used for improving the lather of the composition.

The personal cleansing composition as set out hereinabove may be used for suspending benefits agents selected from the group consisting of hair care and skin care benefit agents, particulates, particles, preferably silica and titanium oxide, microcapsules, oils, droplets, pigments, opacifiers, pearlescent agents, feel modifiers, oil absorbers, skin protectants, matting agents, friction enhancers, slip agents, conditioning agents, exfoliants, odor absorbers, or cleaning enhancers, and mixtures thereof.

The personal cleansing composition can advantageously provide relatively improved ecotoxic or ecologically friendly environmental profile.

The personal cleansing composition can help to provide good esthetic properties such as good foam, and is thick and creamy in texture, is silky to the touch and affords conditioning.

### TEST METHODS

It is understood that the Test Methods that are disclosed in the Test Methods Section of the present application should be used to determine the respective values of the parameters of Applicants' invention as such invention is described and claimed herein.

### Cone/Plate Viscosity Measurement

The viscosity of the personal cleansing composition is measured by a Cone/Plate Brookfield DV12T, by Brookfield Engineering Laboratories, Stoughton, MA. The cone used (Spindle CPA-41z) has a diameter of 24 mm and 3° angle. The viscosity is determined using a steady state flow experiment at constant shear rate of 2 s⁻¹ and at temperature of 26.5 °C. The sample size is 2.5 mL.

### Product pH Measurement

First, calibrate the Metler TOLEDO pH meter. Do this by turning on the pH meter and waiting for 30 seconds. Then, take the electrode out of the storage solution, rinse the electrode with reversed osmosis (RO) water, and carefully wipe the electrode with a scientific cleaning wipe, such as a Kimwipe^{®}. Submerse the electrode in the pH 4 buffer and press the calibrate button. Wait until the pH icon stops flashing. Rinse the electrode with RO water and carefully wipe the electrode with a scientific cleaning wipe. Then submerse the electrode into the pH 7 buffer and wait until the pH icon stops flashing. Rinse the electrode with RO water and carefully wipe with a scientific cleaning wipe. Then, submerse the electrode into the pH 9 buffer and wait until the pH icon stops flashing. Rinse the electrode with distilled RO water and carefully wipe with a scientific cleaning wipe. Now the pH meter is calibrated and can be used to test the pH of a solution.

### EXAMPLES

The following examples further describe and demonstrate embodiments within the scope of the present invention. The examples are given solely for the purpose of illustration and are not to be construed as limitations of the present invention, Where applicable, ingredients are identified by chemical or CTFA name, or otherwise defined below.

The following examples were prepared:

### Compositions (wt.%)

| Ingredients (wt.%) | C. Ex. 1 | C. Ex. 2 | C. Ex. 3 | C. Ex. 4 | Ex. 1 | Ex. 2 |
|---|---|---|---|---|---|---|
| Sodium cocoyl isethionate¹ | 2.00 | 2.00 | 2.00 | 2.00 | 2.00 | 2.50 |
| Sodium lauroyl sarcosinate² | 4.30 | 4.30 | 4.30 | 4.30 | 2.50 | 2.50 |
| Cocamidopropyl betaine³ | 7.75 | 7.75 | 7.70 | 9.00 | 9.00 | 9.00 |
| Sodium salicylate⁶ | 0.40 | 0.40 | 0.45 | 0.40 | 0.40 | 0.40 |
| Sodium benzoate⁷ | 0.45 | 0.45 | 0.45 | 0.45 | 0.45 | 0.45 |
| Citric acid powder⁸ | q.s. pH 5.00 | q.s. pH 6.10 | q.s. pH 5.65 | q.s. pH 5.65 | q.s. pH 5.65 | q.s. pH 5.65 |
| Fragrance | 1.0 | 1.0 | 1.0 | 1.0 | 1.0 | 1.0 |
| Sodium chloride | - | - | 4.00 | 4.00 | 4.00 | 4.00 |
| water | Balance to 100 | Balance to 100 | Balance to 100 | Balance to 100 | Balance to 100 | Balance to 100 |
| | | | | | | |
| Ratio betaine:sarcosinate | 1.80 | 1.80 | 1.79 | 2.09 | 3.60 | 3.60 |
| Ratio sarcosinate:isethionate | 2.15 | 2.15 | 2.15 | 2.15 | 1.25 | 1.00 |
| | | | | | | |
| Initial viscosity (cPs) | 1031 | 50 | 1019 | 1449 | 1817 | 2591 |
| Viscosity after 3 weeks at 60°C (cPs) | 610 | 120 | - | - | - | - |
| Stability results | Phase separation | No Separation | No Separation | No Separation | No Separation | No Separation |

### Definitions of Components

*1 Sodium cocoyl isethionate; Supplier Clariant
*2 Sodium lauroyl sarcosinate; Supplier Tinci
*3 Cocamidopropyl Betaine; Supplier Tinci
*6 Sodium salicylate; Supplier JQC Huayn Pharmaceutical Co Ltd.
*7 Sodium benzoate; Supplier Wuhan Youji Industries
*8 Citric acid powder; Supplier Yixing Union Biochemical
q.s.: sufficient quantity

### Results:

### Phase stability and enhanced viscosity

The personal cleansing composition shall have an initial viscosity from 1.5 Pa.s (1500 cPs) and below 15 Pa.s (15000 cPs).

C. Ex. 1 is a personal cleansing composition comprising sodium cocoyl isethionate, sodium lauroyl sarcosinate and cocamidopropyl betaine at a pH 5.0. After 3 weeks at 60°C, phase separation has been observed with a significant drop of the initial viscosity.

The top floating layer is a lamellar phase layer which is relatively highly ordered and comprises the above surfactants with relatively less water. As their density is lower, the lamellar phase floats and is the upper layer. The micellar phase is the lower layer.

The free fatty acids originating from sodium cocoyl isethionate and sodium lauroyl sarcosinate due to the respective hydrolysis of the anionic surfactants may contribute for the phase transition and phase separation from a micellar phase to a lamellar phase.

Initially, the hydrolysis of sodium cocoyl isethionate and sodium lauroyl sarcosinate causes the viscosity to increase due to the elongation of the wormlike micelles. After the generation of a certain amount of free fatty acid, however, the wormlike and spherical micelles transition from being wormlike micelles to bigger, more complex aggregates, such as disc-like micelles or gel networks. At that moment, these structures decrease the number of entanglements, reduce the resistance to flow, and increase fluidity of the system, resulting in a viscosity drop. A lamellar phase separated from a micellar phase.

When pH is increased from 5.0 to 6.1 in C. Ex. 2, the kinetics of the hydrolysis of sodium cocoyl isethionate and sodium lauroyl sarcosinate have been slowed down. No phase separation was observed. However, still the rheological properties of the personal cleansing composition was not satisfactory to be dispensed to the consumer.

In C. Ex. 3 versus C. Ex. 2, pH has been settled to 5.65 with a level of 4 wt.% of sodium chloride. The initial viscosity of C. Ex. 3 is better than the one of C. Ex. 2 but is still similar as the one of C. Ex. 1, which is not satisfying.

When increasing the level of cocamidopropyl betaine from 7.70 wt.% to 9.0 wt.% between C. Ex. 3 and C. Ex. 4 at a constant level of sodium chloride and at pH of 5.65, the initial viscosity has increased but still not enough as being below 1.5 Pa.s (1500 cPs).

Ex. 1 shows than when the level of sodium lauroyl sarcosinate is decreased from 4.3 wt.% (C. Ex. 4) to 2.5 wt.% (Ex. 1), the initial viscosity has reached 1.81 Pa.s (1817 cPs), which is a desired result.

Ex. 2 showed that the slight increase of sodium cocoyl isethionate from 2.00 wt.% (Ex. 1) to 2.50 wt.% (Ex. 2) could lead to reach the desired enhanced viscosity at 2.59 Pa.s (2591 cPs).

Hence, it has been found that a personal cleansing composition could reach an initial viscosity of 1.8 Pa.s and even further of 2.6 Pa.s when comprising: a) a surfactant system, wherein the surfactant system comprises: i) from 1.75% to 2.75%, preferably from 2.0% to 2.5%, most preferably from 2.35% to 2.5%, of a fatty acyl isethionate surfactant by weight of the composition such as sodium cocoyl isethionate; ii) from 1.75% to 3.0%, most preferably from 2.35% to 2.5%, of a fatty acyl sarcosinate surfactant by weight of the composition such as sodium lauroyl sarcosinate; iii) from 7.75% to 9.75%, most preferably from 9.0% to 9.25% of a zwitterionic surfactant by weight of composition, wherein the zwitterionic surfactant comprises a betaine such as cocamidopropyl betaine; b) wherein the pH is from 5.5 to 7, preferably from 5.6 to 6.5, more preferably from 5.65 to 6.0; and wherein the composition is substantially free of from alkyl sulfate and alkyl ether sulfate type of surfactants.

Especially, the surfactant ratios can be optimized to enable building and improving the rheology profile of the personal cleansing composition. For this, the weight ratio of betaine to fatty acyl sarcosinate surfactant may be above 2.5:1, or from 3.4:1 to 3.8:1, preferably from 3.45:1 to 3.7:1, more preferably from 3.5:1 to 3.65:1.

Also, or alternatively, the weight ratio of fatty acyl sarcosinate surfactant to fatty acyl isethionate may be above 0.5:1, or from 1.75:1 to 0.65:1, preferably from 1.25:1 to 0.75:1, more preferably from 1.2:1 to 0.95:1.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A personal cleansing composition comprising:
a) a surfactant system, wherein the surfactant system comprises:
(i) from 1.75% to 2.75%, preferably from 2.0% to 2.5%, more preferably from 2.25% to 2.5%, most preferably from 2.35% to 2.5%, of a fatty acyl isethionate surfactant by weight of the composition;
(ii) from 1.75% to 3.0%, preferably from 2.0% to 2.5%, more preferably from 2.25% to 2.5%, most preferably from 2.35% to 2.5%, of a fatty acyl sarcosinate surfactant by weight of the composition;
(iii)from 7.75% to 9.75%, preferably from 8.0% to 9.5%, more preferably from 8.75% to 9.5%, most preferably from 9.0% to 9.25% of a zwitterionic surfactant by weight of composition, wherein the zwitterionic surfactant comprises a betaine;
b) wherein the pH is from 5.5 to 7, preferably from 5.6 to 6.5, more preferably from 5.65 to 6.0; and
c) wherein the composition comprises less than 1.5% of alkyl sulfate and/or alkyl ether sulfate type of surfactants by weight of the composition.

2. The personal cleansing composition of claim **1,** wherein the weight ratio of betaine to fatty acyl sarcosinate surfactant is above 2.5:1, or from 3.4:1 to 3.8:1, preferably from 3.45:1 to 3.7:1, more preferably from 3.5:1 to 3.65:1.

3. The personal cleansing composition of any of the preceding claims, wherein the weight ratio of fatty acyl sarcosinate surfactant to fatty acyl isethionate is above 0.5:1, or from 0.65:1 tol.75:1, preferably from 0.75:1 to 1.25:1, more preferably from 0.95:1 to 1.2:1.

4. The personal cleansing composition of any of the preceding claims, wherein the fatty acyl isethionate surfactant is an isethionate according to the general Formula (I): 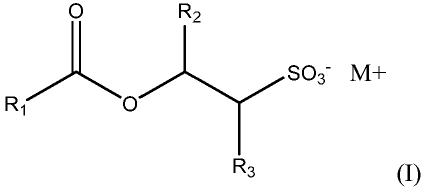 wherein R₁ is a saturated or unsaturated, straight or branched, alkyl or alkenyl chain with from 6 to 30 carbon atoms, preferably from 8 to 22 carbon atoms, more preferably from 9 to 18 carbon atoms, R₂ and R₃ are each independently H or (C₁-C₄) alkyl, and M⁺ is an alkali metal, preferably lithium, sodium, potassium; or M⁺ is an alkali-earth metal, preferably magnesium; or M⁺ is an ammonium or a substituted ammonium cation; or preferably wherein R₁ is a saturated or unsaturated, straight or branched alkyl or alkenyl, preferably an alkyl chain with from 6 to 30 carbon atoms, preferably from 8 to 22 carbon atoms, more preferably from 9 to 18 carbon atoms, R₂ and R₃ are H, and M⁻ is an alkali metal, preferably sodium, potassium; or M⁺ is an ammonium cation; or more preferably wherein R₁ is a saturated or unsaturated, straight or branched alkyl chain with from 9 to 18 carbon atoms, R₂ and R₃ are H, and M⁺ is sodium or an ammonium cation.

5. The personal cleansing composition of claim 4, wherein the fatty acyl isethionate surfactant is selected from the group consisting of sodium lauroyl isethionate, sodium lauroyl methyl isethionate, sodium oleoyl isethionate, sodium oleoyl methyl isethionate, sodium stearoyl isethionate, sodium stearoyl methyl isethionate, sodium myristoyl isethionate, sodium myristoyl methyl isethionate, sodium palmitoyl isethionate, sodium palmitoyl methyl isethionate, sodium cocoyl isethionate, sodium cocoyl methyl isethionate, a blend of stearic acid and sodium cocoyl isethionate, ammonium cocoyl isethionate, ammonium cocoyl methyl isethionate, and mixtures thereof; preferably wherein the fatty acyl isethionate surfactant is selected from the group consisting of sodium lauroyl isethionate, sodium cocoyl isethionate, ammonium cocoyl isethionate, and mixtures thereof; more preferably wherein the fatty acyl isethionate surfactant is selected from the group consisting of sodium cocoyl isethionate, ammonium cocoyl isethionate, and mixtures thereof; even more preferably wherein the fatty acyl isethionate surfactant comprises sodium cocoyl isethionate.

6. The personal cleansing composition of any of the preceding claims, wherein the fatty acyl sarcosinate surfactant is a sarcosinate according to the general formula (II): 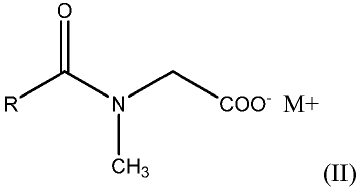 wherein R is a saturated or unsaturated, straight or branched or alkenyl, preferably alkyl chain with 7 to 17 carbon atoms, preferably with 9 to 13 carbon atoms and M⁺ is H, a sodium, potassium or ammonium cation.

7. The personal cleansing composition of claim 6, wherein the fatty acyl sarcosinate surfactant is selected from the group consisting of sodium lauroyl sarcosinate, sodium cocoyl sarcosinate, sodium myristoyl sarcosinate, TEA-cocoyl sarcosinate, ammonium cocoyl sarcosinate, ammonium lauroyl sarcosinate, dimer dilinoleyl bis-lauroyl glutamate/lauroyl sarcosinate, lauroyl sarcosinate, isopropyl lauroyl sarcosinate, potassium cocoyl sarcosinate, potassium lauroyl sarcosinate, sodium oleoyl sarcosinate, sodium palmitoyl sarcosinate, TEA-lauroyl sarcosinate, TEA-oleoyl sarcosinate, TEA-palm kernel sarcosinate, and mixtures thereof; preferably wherein the fatty acyl sarcosinate surfactant is selected from the group consisting of sodium lauroyl sarcosinate, sodium myristoyl sarcosinate, sodium cocoyl sarcosinate, and mixtures thereof; more preferably wherein the fatty acyl sarcosinate surfactant comprises sodium lauroyl sarcosinate.

8. The personal cleansing composition of any of the preceding claims, wherein the betaine is selected from the group consisting of cocamidopropyl betaine, lauramidopropyl betaine, coco betaine, and mixtures thereof; preferably wherein the betaine comprises cocamidopropyl betaine.

9. The personal cleansing composition of claim 8, wherein the composition comprises cocamidopropyl betaine and sodium lauroyl sarcosinate at a weight ratio betaine to sarcosinate from 3.4:1 to 3.8:1, preferably from 3.45:1 to 3.7:1, more preferably from 3.5:1 to 3.65:1.

10. The personal cleansing composition of any ones of claims 7 to 9, wherein the composition comprises sodium lauroyl sarcosinate and sodium cocoyl isethionate at a weight ratio sarcosinate to isethionate from 1:0.65 to 1.75:1, preferably from 1:0.75 to 1.25:1, more preferably from 1:0.95 to 1.2:1.

11. The personal cleansing composition of any of the preceding claims, wherein the personal cleansing composition further comprises from 0.05% to 5%, preferably from 1% to 5%, more preferably from 2% to 5%, even more preferably from 2.75% to 4% of an electrolyte by weight of the composition, wherein the electrolyte is selected from the group of sodium or potassium citrate, calcium chloride, calcium bromide, zinc chloride, barium chloride, calcium nitrate, potassium chloride, sodium chloride, potassium iodide, sodium bromide, ammonium bromide, sodium sulfate, and mixtures thereof; preferably wherein the electrolyte is selected from the group of sodium or potassium citrate, calcium chloride, potassium chloride, sodium chloride, and mixtures thereof; most preferably wherein the personal cleansing composition further comprises from 3% to 4% of an electrolyte by weight of the composition, wherein the electrolyte is sodium chloride.

12. The personal cleansing composition of any of the preceding claims, wherein the personal cleansing composition further comprises from 0.01 wt.% to 2 wt.% of a fragrance component by weight of the composition; preferably from 0.1 wt.% to 1.75 wt.% of a fragrance component by weight of the composition; more preferably from 0.5 wt.% to 1.6 wt.% of a fragrance component by weight of the composition; even more preferably from 0.8 wt.% to 1.0 wt.% of a fragrance component by weight of the composition.

13. The personal cleansing composition of any of the preceding claims, wherein the composition comprises from 0.01% to 1.0%, preferably from 0.02% to 0.4%, more preferably from 0.05% to 0.2%, most preferably from 0.05% to 0.1% of a preservative by weight of the composition; preferably wherein the preservative includes a salicylate salt and a benzoate salt, wherein a total amount of the salicylate salt and the benzoate salt is from 0.2% to 0.9%, preferably from 0.5% to 0.85%, more preferably from 0.75% to 0.85%, by weight of the composition.

14. The personal cleansing composition of claim 13, wherein the weight ratio of the salicylate salt to the benzoate salt is from 1:1.10 to 1:1.20, preferably from 1:1.125 to 1:1.175.

15. A method of building a consistent viscosity of a stable personal cleansing composition comprising the step of forming a personal cleansing composition of any of the preceding claims.

## Patentansprüche

1. Körperreinigungszusammensetzung, umfassend:
a) ein Tensidsystem, wobei das Tensidsystem umfasst:
(i) von zu 1,75 % bis 2,75 %, vorzugsweise von zu 2,0 % bis 2,5 %, mehr bevorzugt von zu 2,25 % bis 2,5 %, am meisten bevorzugt von zu 2,35 % bis 2,5 %, ein Fatty-Acyl-Isethionattensid, bezogen auf das Gewicht der Zusammensetzung;
(ii) von zu 1,75 % bis 3,0 %, vorzugsweise von zu 2,0 % bis 2,5 %, mehr bevorzugt von zu 2,25 % bis 2,5 %, am meisten bevorzugt von zu 2,35 % bis 2,5 %, ein Fatty-Acyl-Sarcosinattensid, bezogen auf das Gewicht der Zusammensetzung;
(iii) von zu 7,75 % bis 9,75 %, vorzugsweise von zu 8,0 % bis 9,5 %, mehr bevorzugt von zu 8,75 % bis 9,5 %, am meisten bevorzugt von zu 9,0 % bis 9,25 %, ein zwitterionisches Tensid, bezogen auf das Gewicht der Zusammensetzung, wobei das zwitterionische Tensid ein Betain umfasst;
b) wobei der pH-Wert von 5,5 bis 7, vorzugsweise von 5,6 bis 6,5, mehr bevorzugt von 5,65 bis 6,0 beträgt; und
c) wobei die Zusammensetzung zu weniger als 1,5 % Tenside eines Alkylsulfat- und/oder Alkylethersulfattyps, bezogen auf das Gewicht der Zusammensetzung, umfasst.

2. Körperreinigungszusammensetzung nach Anspruch 1, wobei das Gewichtsverhältnis von Betain zu Fatty-Acyl-Sarcosinattensid über 2,5 : 1 oder von 3,4 : 1 bis 3,8 : 1, vorzugsweise von 3,45 : 1 bis 3,7 : 1, mehr bevorzugt von 3,5 : 1 bis 3,65 : 1, beträgt.

3. Körperreinigungszusammensetzung nach einem der vorstehenden Ansprüche, wobei das Gewichtsverhältnis von Fatty-Acyl-Sarcosinattensid zu Fatty-Acyl-Isethionat über 0,5 : 1 oder von 0,65 : 1 bis 1,75 : 1, vorzugsweise von 0,75 : 1 bis 1,25 : 1, mehr bevorzugt von 0,95 : 1 bis 1,2 : 1, beträgt.

4. Körperreinigungszusammensetzung nach einem der vorstehenden Ansprüche, wobei das Fatty-Acyl-Isethionattensid ein Isethionat nach der allgemeinen Formel (I) ist: 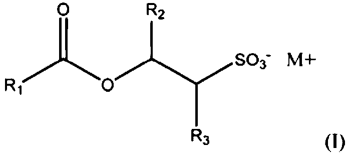 wobei R₁ eine gesättigte oder ungesättigte, geradkettige oder verzweigte, Alkyl- oder Alkenylkette mit von 6 bis 30 Kohlenstoffatomen, vorzugsweise von 8 bis 22 Kohlenstoffatomen, mehr bevorzugt von 9 bis 18 Kohlenstoffatomen, ist, R₂ und R₃ jeweils unabhängig voneinander H oder (C₁-C₄)-Alkyl sind und M⁺ ein Alkalimetall, vorzugsweise Lithium, Natrium, Kalium, ist; oder M⁺ ein Erdalkalimetall, vorzugsweise Magnesium, ist; oder M⁺ ein Ammonium- oder ein substituiertes Ammoniumkation ist; oder wobei R₁ vorzugsweise ein gesättigtes oder ungesättigtes, geradkettiges oder verzweigtes Alkyl oder Alkenyl, vorzugsweise eine Alkylkette mit von 6 bis 30 Kohlenstoffatomen, vorzugsweise von 8 bis 22 Kohlenstoffatomen, mehr bevorzugt von 9 bis 18 Kohlenstoffatomen, ist, R₂ und R₃ H sind und M⁺ ein Alkalimetall, vorzugsweise Natrium, Kalium, ist; oder M⁺ ein Ammoniumkation ist; oder wobei R₁ mehr bevorzugt eine gesättigte oder ungesättigte, geradkettige oder verzweigte Alkylkette mit von 9 bis 18 Kohlenstoffatomen ist, R₂ und R₃ H sind und M⁺ Natrium oder ein Ammoniumkation ist.

5. Körperreinigungszusammensetzung nach Anspruch 4, wobei das Fatty-Acyl-Isethionattensid ausgewählt ist aus der Gruppe, bestehend aus Natriumlauroylisethionat, Natriumlauroylmethylisethionat, Natriumoleoylisethionat, Natriumoleoylmethylisethionat, Natriumstearoylisethionat, Natriumstearoylmethylisethionat, Natriummyristoylisethionat, Natriummyristoylmethylisethionat, Natriumpalmitoylisethionat, Natriumpalmitoylmethylisethionat, Natriumcocoylisethionat, Natriumcocoylmethylisethionat, einem Gemisch aus Stearinsäure und Natriumcocoylisethionat, Ammoniumcocoylisethionat, Ammoniumcocoylmethylisethionat und Mischungen davon; wobei das Fatty-Acyl-Isethionattensid vorzugsweise ausgewählt ist aus der Gruppe, bestehend aus Natriumlauroylisethionat, Natriumcocoylisethionat, Ammoniumcocoylisethionat und Mischungen davon; wobei das Fatty-Acyl-Isethionattensid mehr bevorzugt ausgewählt ist aus der Gruppe, bestehend aus Natriumcocoylisethionat, Ammoniumcocoylisethionat und Mischungen davon; wobei das Fatty-Acyl-Isethionattensid noch mehr bevorzugt Natriumcocoylisethionat umfasst.

6. Körperreinigungszusammensetzung nach einem der vorstehenden Ansprüche, wobei das Fatty-Acyl-Sarcosinattensid ein Sarcosinat nach der allgemeinen Formel (II) ist: 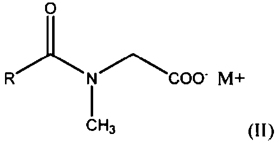 wobei R ein gesättigtes oder ungesättigtes, geradkettiges oder verzweigtes oder Alkenyl ist, vorzugsweise eine Alkylkette mit 7 bis 17 Kohlenstoffatomen, vorzugsweise mit 9 bis 13 Kohlenstoffatomen und M⁺ H, ein Natrium, ein Kalium oder ein Ammoniumkation ist.

7. Körperreinigungszusammensetzung nach Anspruch 6, wobei das Fatty-Acyl-Sarcosinattensid ausgewählt ist aus der Gruppe, bestehend aus Natriumlauroylsarcosinat, Natriumcocoylsarcosinat, Natriummyristoylsarcosinat, TEA-Cocoylsarcosinat, Ammoniumcocoylsarcosinat, Ammoniumlauroylsarcosinat, Dimerdilinoleylbislauroylglutamat/Lauroylsarcosinat, Lauroylsarcosinat, Isopropyllauroylsarcosinat, Kaliumcocoylsarcosinat, Kaliumlauroylsarcosinat, Natriumoleoylsarcosinat, Natriumpalmitoylsarcosinat, TEA-Lauroylsarcosinat, TEA-Oleoylsarcosinat, TEA-Palmkernsarcosinat und Mischungen davon; wobei das Fatty-Acyl-Sarcosinattensid vorzugsweise ausgewählt ist aus der Gruppe, bestehend aus Natriumlauroylsarcosinat, Natriummyristoylsarcosinat, Natriumcocoylsarcosinat und Mischungen davon; wobei das Fatty-Acyl-Sarcosinattensid mehr bevorzugt Natriumlauroylsarcosinat umfasst.

8. Körperreinigungszusammensetzung nach einem der vorstehenden Ansprüche, wobei das Betain ausgewählt ist aus der Gruppe, bestehend aus Cocamidopropylbetain, Lauramidopropylbetain, Cocobetain und Mischungen davon; wobei das Betain vorzugsweise Cocamidopropylbetain umfasst.

9. Körperreinigungszusammensetzung nach Anspruch 8, wobei die Zusammensetzung Cocamidopropylbetain und Natriumlauroylsarcosinat in einem Gewichtsverhältnis von Betain zu Sarcosinat von 3,4 : 1 bis 3,8 : 1, vorzugsweise von 3,45 : 1 bis 3,7 : 1, mehr bevorzugt von 3,5:1 bis 3,65 : 1, umfasst.

10. Körperreinigungszusammensetzung nach einem der Ansprüche 7 bis 9, wobei die Zusammensetzung Natriumlauroylsarcosinat und Natriumcocoylisethionat in einem Gewichtsverhältnis von Sarcosinat zu Isethionat von 1 : 0,65 bis 1,75 : 1, vorzugsweise von 1 : 0,75 bis 1,25 : 1, mehr bevorzugt von 1 : 0,95 bis 1,2 : 1, umfasst.

11. Körperreinigungszusammensetzung nach einem der vorstehenden Ansprüche, wobei die Körperreinigungszusammensetzung ferner von zu 0,05 % bis 5 %, vorzugsweise von zu 1 % bis 5 %, mehr bevorzugt von zu 2 % bis 5 %, noch mehr bevorzugt von zu 2,75 % bis 4 %, einen Elektrolyten, bezogen auf das Gewicht der Zusammensetzung, umfasst, wobei der Elektrolyt ausgewählt ist aus der Gruppe, bestehend aus Natrium- oder Kaliumcitrat, Calciumchlorid, Calciumbromid, Zinkchlorid, Bariumchlorid, Calciumnitrat, Kaliumchlorid, Natriumchlorid, Kaliumiodid, Natriumbromid, Ammoniumbromid, Natriumsulfat und Mischungen davon; wobei der Elektrolyt vorzugsweise ausgewählt ist aus der Gruppe, bestehend aus Natrium- oder Kaliumcitrat, Calciumchlorid, Kaliumchlorid, Natriumchlorid und Mischungen davon; wobei die Körperreinigungszusammensetzung ferner am meisten bevorzugt von zu 3 % bis 4 % einen Elektrolyten, bezogen auf das Gewicht der Zusammensetzung, umfasst, wobei der Elektrolyt Natriumchlorid ist.

12. Körperreinigungszusammensetzung nach einem der vorstehenden Ansprüche, wobei die Körperreinigungszusammensetzung ferner von zu 0,01 Gew.-% bis 2 Gew.-% einen Duftstoffbestandteil, bezogen auf das Gewicht der Zusammensetzung; vorzugsweise von zu 0,1 Gew.-% bis 1,75 Gew.-% einen Duftstoffbestandteil, bezogen auf das Gewicht der Zusammensetzung; mehr bevorzugt von zu 0,5 Gew.-% bis 1,6 Gew.-% einen Duftstoffbestandteil, bezogen auf das Gewicht der Zusammensetzung; noch mehr bevorzugt von zu 0,8 Gew.-% bis 1,0 Gew.-% einen Duftstoffbestandteil, bezogen auf das Gewicht der Zusammensetzung, umfasst.

13. Körperreinigungszusammensetzung nach einem der vorstehenden Ansprüche, wobei die Zusammensetzung von zu 0,01 % bis 1,0 %, vorzugsweise von zu 0,02 % bis 0,4 %, mehr bevorzugt von zu 0,05 % bis 0,2 %, am meisten bevorzugt von zu 0,05 % bis 0,1 % einen Konservierungsstoff, bezogen auf das Gewicht der Zusammensetzung, umfasst; wobei der Konservierungsstoff vorzugsweise ein Salicylatsalz und ein Benzoatsalz einschließt, wobei eine Gesamtmenge des Salicylatsalzes und des Benzoatsalzes von zu 0,2 % bis 0,9 %, vorzugsweise von zu 0,5 % bis 0,85 %, mehr bevorzugt von zu 0,75 % bis 0,85 %, bezogen auf das Gewicht der Zusammensetzung, beträgt.

14. Körperreinigungszusammensetzung nach Anspruch 13, wobei das Gewichtsverhältnis des Salicylatsalzes zu dem Benzoatsalz von 1 : 1,10 bis 1 : 1,20, vorzugsweise von 1 : 1,125 bis 1 : 1,175, beträgt.

15. Verfahren zum Aufbauen einer konsistenten Viskosität einer stabilen Körperreinigungszusammensetzung, umfassend den Schritt eines Ausbildens einer Körperreinigungszusammensetzung nach einem der vorstehenden Ansprüche.

## Revendications

1. Composition d'hygiène personnelle comprenant :
a) un système tensioactif, dans laquelle le système tensioactif comprend :
(i) de 1,75 % à 2,75 %, de préférence de 2,0 % à 2,5 %, plus préférablement de 2,25 % à 2,5 %, le plus préférablement de 2,35 % à 2,5 % d'un tensioactif iséthionate d'acyle gras en poids de la composition ;
(ii) de 1,75 % à 3,0 %, de préférence de 2,0 % à 2,5 %, plus préférablement de 2,25 % à 2,5 %, le plus préférablement de 2,35 % à 2,5 % d'un tensioactif sarcosinate d'acyle gras en poids de la composition ;
(iii) de 7,75 % à 9,75 %, de préférence de 8,0 % à 9,5 %, plus préférablement de 8,75 % à 9,5 %, le plus préférablement de 9,0 % à 9,25 % d'un tensioactif zwitterionique en poids de composition, dans laquelle le tensioactif zwitterionique comprend une bétaïne ;
b) dans laquelle le pH est de 5,5 à 7, de préférence de 5,6 à 6,5, plus préférablement de 5,65 à 6,0 ; et
c) dans laquelle la composition comprend moins de 1,5 % de tensioactifs de type sulfate d'alkyle et/ou sulfate d'éther d'alkyle en poids de la composition.

2. Composition d'hygiène personnelle selon la revendication 1, dans laquelle le rapport de poids de la bétaïne au tensioactif sarcosinate d'acyle gras est supérieur à 2,5:1, ou va de 3,4:1 à 3,8:1, de préférence de 3,45:1 à 3,7:1, plus préférablement de 3,5:1 à 3,65:1.

3. Composition d'hygiène personnelle selon l'une quelconque des revendications précédentes, dans laquelle le rapport de poids du tensioactif sarcosinate d'acyle gras à l'iséthionate d'acyle gras est supérieur à 0,5:1, ou va de 0,65:1 à 1,75:1, de préférence de 0,75:1 à 1,25:1, plus préférablement de 0,95:1 à 1,2:1.

4. Composition d'hygiène personnelle selon l'une quelconque des revendications précédentes, dans laquelle le tensioactif iséthionate d'acyle gras est un iséthionate selon la formule générale (1) : 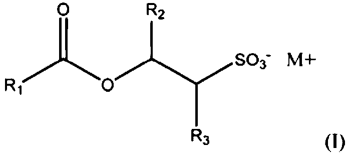 dans laquelle R₁ est une chaîne alkyle ou alcényle saturée ou insaturée, linéaire ou ramifiée, avec de 6 à 30 atomes de carbone, de préférence de 8 à 22 atomes de carbone, plus préférablement de 9 à 18 atomes de carbone, R₂ et R₃ sont chacun indépendamment H ou alkyle en (C₁ à C₄), et M⁺ est un métal alcalin, de préférence lithium, sodium, potassium ; ou M⁺ est un métal alcalino-terreux, de préférence magnésium ; ou M⁺est un cation ammonium ou ammonium substitué ; ou de préférence dans laquelle R₄ est un alkyle ou alcényle saturé ou insaturé, linéaire ou ramifié, de préférence une chaîne alkyle avec de 6 à 30 atomes de carbone, de préférence de 8 à 22 atomes de carbone, plus préférablement de 9 à 18 atomes de carbone, R₂ et R₃ sont H, et M⁺ est un métal alcalin, de préférence sodium, potassium ; ou M⁺ est un cation ammonium ; ou plus préférablement dans laquelle R₁ est une chaîne alkyle saturée ou insaturée, linéaire ou ramifiée avec de 9 à 18 atomes de carbone, R₂ et R₃ sont H, et M⁺ est sodium ou un cation ammonium.

5. Composition d'hygiène personnelle selon la revendication 4, dans laquelle le tensioactif iséthionate d'acyle gras est choisi dans le groupe constitué de lauroyl iséthionate de sodium, lauroyl méthyl iséthionate de sodium, oléoyl iséthionate de sodium, oléoyl méthyl iséthionate de sodium, stéaroyl iséthionate de sodium, stéaroyl méthyl iséthionate de sodium, myristoyl iséthionate de sodium, myristoyl méthyl iséthionate de sodium, palmitoyl iséthionate de sodium, palmitoyl méthyl iséthionate de sodium, cocoyl iséthionate de sodium, cocoyl méthyl iséthionate de sodium, un mélange d'acide stéarique et de cocoyl iséthionate de sodium, cocoyl iséthionate d'ammonium, cocoyl méthyl iséthionate d'ammonium, et mélanges de ceux-ci ; de préférence dans laquelle le tensioactif iséthionate d'acyle gras est choisi dans le groupe constitué de lauroyl iséthionate de sodium, cocoyl iséthionate de sodium, cocoyl iséthionate d'ammonium, et mélanges de ceux-ci ; plus préférablement dans laquelle le tensioactif iséthionate d'acyle gras est choisi dans le groupe constitué de cocoyl iséthionate de sodium, cocoyl iséthionate d'ammonium et mélanges de ceux-ci ; encore plus préférablement dans laquelle le tensioactif iséthionate d'acyle gras comprend le cocoyl iséthionate de sodium.

6. Composition d'hygiène personnelle selon l'une quelconque des revendications précédentes, dans laquelle le tensioactif sarcosinate d'acyle gras est un sarcosinate selon la formule générale (II) : 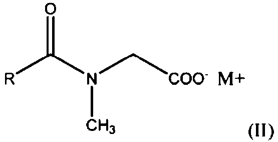 dans laquelle R est un alcényle ou saturé ou insaturé, linéaire ou ramifié, de préférence une chaîne alkyle avec de 7 à 17 atomes de carbone, de préférence avec de 9 à 13 atomes de carbone et M⁺ est **H,** un cation sodium, potassium ou ammonium.

7. Composition d'hygiène personnelle selon la revendication 6, dans laquelle le tensioactif sarcosinate d'acyle gras est choisi dans le groupe constitué de lauroyl sarcosinate de sodium, cocoyl sarcosinate de sodium, myristoyl sarcosinate de sodium, TEA-cocoyl sarcosinate, cocoyl sarcosinate d'ammonium, lauroyl sarcosinate d'ammonium, dimère dilinoleyl bis-lauroyl glutamate/lauroyl sarcosinate, lauroyl sarcosinate, lauroyl sarcosinate d'isopropyle, cocoyl sarcosinate de potassium, lauroyl sarcosinate de potassium, oléoyl sarcosinate de sodium, palmitoyl sarcosinate de sodium, TEA-lauroyl sarcosinate, TEA-oléoyl sarcosinate, TEA-palm kernel sarcosinate, et mélanges de ceux-ci ; de préférence, dans laquelle le tensioactif sarcosinate d'acyle gras est choisi dans le groupe constitué de lauroyl sarcosinate de sodium, myristoyl sarcosinate de sodium, cocoyl sarcosinate de sodium et mélanges de ceux-ci ; plus préférablement dans laquelle le tensioactif sarcosinate d'acyle gras comprend le lauroyl sarcosinate de sodium.

8. Composition d'hygiène personnelle selon l'une quelconque des revendications précédentes, dans laquelle la bétaïne est choisie dans le groupe constitué de bétaïne de cocamidopropyle, bétaïne de lauramidopropyle, coco bétaïne et mélanges de celles-ci ; de préférence dans laquelle la bétaïne comprend la bétaïne de cocamidopropyle.

9. Composition d'hygiène personnelle selon la revendication 8, dans laquelle la composition comprend de la bétaïne de cocamidopropyle et du lauroyl sarcosinate de sodium dans un rapport de poids de la bétaïne au sarcosinate de 3,4:1 à 3,8:1, de préférence de 3,45:1 à 3,7:1, plus préférablement de 3,5:1 à 3,65:1.

10. Composition d'hygiène personnelle selon l'une quelconque des revendications 7 à 9, dans laquelle la composition comprend du lauroyl sarcosinate de sodium et du cocoyl iséthionate de sodium dans un rapport de poids de sarcosinate à iséthionate de 1:0,65 à 1,75:1, de préférence de 1:0,75 à 1,25:1, plus préférablement de 1:0,95 à 1,2:1.

11. Composition d'hygiène personnelle selon l'une quelconque des revendications précédentes, dans laquelle la composition d'hygiène personnelle comprend en outre de 0,05 % à 5 %, de préférence de 1 % à 5 %, plus préférablement de 2 % à 5 %, encore plus préférablement de 2,75 % à 4 % d'un électrolyte en poids de la composition, dans laquelle l'électrolyte est choisi dans le groupe de citrate de sodium ou de potassium, chlorure de calcium, bromure de calcium, chlorure de zinc, chlorure de baryum, nitrate de calcium, chlorure de potassium, chlorure de sodium, iodure de potassium, bromure de sodium, bromure d'ammonium, sulfate de sodium et mélanges de ceux-ci ; de préférence dans laquelle l'électrolyte est choisi dans le groupe de citrate de sodium ou de potassium, chlorure de calcium, chlorure de potassium, chlorure de sodium et mélanges de ceux-ci ; le plus préférablement dans laquelle la composition d'hygiène personnelle comprend en outre de 3 % à 4 % d'un électrolyte en poids de la composition, dans laquelle l'électrolyte est chlorure de sodium.

12. Composition d'hygiène personnelle selon l'une quelconque des revendications précédentes, dans laquelle la composition d'hygiène personnelle comprend en outre de 0,01 % en poids à 2 % en poids d'un composant de parfum par rapport au poids de la composition ; de préférence de 0,1 % en poids à 1,75 % en poids d'un composant de parfum par rapport au poids de la composition ; plus préférablement de 0,5 % en poids à 1,6 % en poids d'un composant de parfum par rapport au poids de la composition ; encore plus préférablement de 0,8 % en poids à 1,0 % en poids d'un composant de parfum par rapport au poids de la composition.

13. Composition d'hygiène personnelle selon l'une quelconque des revendications précédentes, dans laquelle la composition comprend de 0,01 % à 1,0 %, de préférence de 0,02 % à 0,4 %, plus préférablement de 0,05 % à 0,2 %, le plus préférablement de 0,05 % à 0,1 % d'un agent de conservation en poids de la composition ; de préférence dans laquelle l'agent de conservation comporte un sel de salicylate et un sel de benzoate, dans laquelle une quantité totale de sel de salicylate et de sel de benzoate est comprise entre 0,2 % et 0,9 %, de préférence entre 0,5 % et 0,85 %, plus préférablement entre 0,75 % et 0,85 %, en poids de la composition.

14. Composition d'hygiène personnelle selon la revendication 13, dans laquelle le rapport de poids du sel de salicylate au sel de benzoate est de 1:1,10 à 1:1,20, de préférence de 1:1,125 à 1:1,175.

15. Procédé pour obtenir une viscosité constante d'une composition stable d'hygiène personnelle, comprenant l'étape de formation d'une composition d'hygiène personnelle selon l'une quelconque des revendications précédentes.
